# EUROPEAN PATENT APPLICATION

(11) **EP 4 502 614 A1**
(43) Date of publication of application: **05.02.2025**
(21) Application number: 23780738.3
(22) Date of filing: 29.03.2023
(51) Int. Cl.: G01N 35/00

(54) **SPECIMEN PROCESSING SYSTEM**

(30) Priority: 30.03.2022 JP 2022055892
(71) Applicant: Sekisui Medical Co., Ltd., Tokyo 103-0027 (JP)
(72) Inventor: OGASAWARA, Kosuke, Tokyo 103-0027 (JP); KAWABE, Toshiki, Tokyo 103-0027 (JP); IIJIMA, Yumi, Tokyo 103-0027 (JP)
(74) Representative: Maiwald GmbH
(86) International application number: PCT/JP2023/012906
(87) International publication number: WO 2023/190730

(57) **Abstract**

In a specimen processing system (10), plural pieces of accuracy control data that is time series data of an accuracy control value obtained by analyzing an accuracy control sample by an analysis unit (22) is stored in an accuracy control measurement information database (74). Then, the display control unit (88) simultaneously displays the accuracy control data of plural accuracy control samples of the same type and different production lot numbers on a touch display (80) based on the accuracy control data stored in the accuracy control measurement information database (74).

## Description

### Technical Field

The present disclosure relates to a specimen processing system.

### Background Art

Japanese Patent Application Laid-Open (JP-A) No. 2012-233799 describes an invention related to a specimen analyzer. In this specimen analyzer, a plurality of accuracy control graphs selected as display targets among a plurality of pieces of accuracy control data of accuracy control samples is simultaneously displayed on a display unit. Then, when the number of accuracy control values included in the same unit period is different among the plurality of accuracy control graphs, the same time series range is assigned to the unit period in the accuracy control graphs, and the accuracy control values in the unit period corresponding to the time series range are plotted. Thus, it is possible to easily compare the accuracy control data of a plurality of accuracy control samples having different measurement frequencies.

### SUMMARY OF INVENTION

### Technical Problem

Incidentally, the concentration of a predetermined measurement target substance in the accuracy control sample differs for each production lot, and thus, when a production lot number of an accuracy control sample currently used for accuracy control is different from a production lot number of an accuracy control sample scheduled to be used for accuracy control, it is preferable that tendencies of accuracy control values of an accuracy control sample to be used for future accuracy control can be grasped in advance.

In view of the above fact, an object of the disclosure is to provide a specimen processing system capable of grasping in advance tendencies of accuracy control values of an accuracy control sample to be used for future accuracy control.

### Solution to Problem

A specimen processing system according to a first aspect includes an analysis unit that analyzes a component in a specimen, an accuracy control information storage unit that stores a plurality of pieces of accuracy control data that is time series data of an accuracy control value obtained by analyzing an accuracy control sample by the analysis unit, and a display control unit that causes a display unit to display the accuracy control data, in which the display control unit simultaneously displays the accuracy control data of a plurality of accuracy control samples of a same type and different production lot numbers on the display unit based on the accuracy control data stored in the accuracy control information storage unit.

The specimen processing system according to the first aspect includes an analysis unit, and a component in a specimen is analyzed by the analysis unit.

Incidentally, in order to ensure the reliability of the specimen processing system, it is necessary to measure the accuracy control sample containing the measurement target substance in the specimen, that is, perform the accuracy control in the analysis unit before and after the analysis of the specimen.

Then, the concentration of the predetermined measurement target substance in the accuracy control sample differs for each production lot, and thus, when the production lot number of the accuracy control sample currently used for accuracy control is different from the production lot number of the accuracy control sample scheduled to be used for accuracy control, it is preferable that tendencies of the accuracy control values of the accuracy control sample to be used for the future accuracy control can be grasped in advance.

Here, in the present aspect, the accuracy control information storage unit stores a plurality of pieces of accuracy control data that is time series data of the accuracy control value obtained by analyzing the accuracy control sample by the analysis unit, and the accuracy control data is displayed on the display unit by the display control unit.

Specifically, the display control unit simultaneously displays the accuracy control data of a plurality of accuracy control samples of the same type and different production lot numbers on the display unit based on the accuracy control data stored in the accuracy control information storage unit. Thus, in the present aspect, the user can compare the accuracy control data of a plurality of accuracy control samples of the same type and different production lot numbers.

For example, the user can compare the accuracy control sample currently used for accuracy control with the accuracy control sample scheduled to be used for accuracy control and currently used and an accuracy control sample of a different production lot number.

A specimen processing system according to a second aspect is the specimen processing system according to the first aspect, in which the accuracy control data includes a single or a plurality of the accuracy control values obtained on different analysis dates, and the display control unit displays an accuracy control graph obtained by plotting the accuracy control values as the accuracy control data.

In the specimen processing system according to the second aspect, the accuracy control data includes a single or a plurality of the accuracy control values obtained on different analysis dates, and the display control unit displays an accuracy control graph obtained by plotting the accuracy control values on the display unit as the accuracy control data. Thus, in the present aspect, the user can observe changes in the accuracy control values on a plurality of analysis days by viewing the accuracy control data.

A specimen processing system according to a third aspect is the specimen processing system according to the second aspect, in which the display control unit plots a single or a plurality of the accuracy control values obtained on the analysis dates for each of a plurality of plot areas divided in the accuracy control graph in time series order, and a size in a time series direction of the plurality of plot areas is set to a size at which it is possible to ensure a predetermined interval for an interval for each of plots in a plot area having a largest number of the plots of the accuracy control values.

According to the specimen processing system according to the third aspect, by plotting the accuracy control values obtained on the analysis date in time series order for each of the plurality of plot areas, it is possible to observe changes in the accuracy control values in more detail.

By setting the size of the plot area in the time series direction to a size in which the interval between the plots in the plot area having the largest number of plots of the accuracy control values can ensure a predetermined interval, it is possible to ensure the visibility of the accuracy control graph.

A specimen processing system according to a fourth aspect is the specimen processing system according to the third aspect, in which a number of the plots of the accuracy control values displayed in the plot area is set to from 15 to 125.

In the specimen processing system according to the fourth aspect, the number of plots of the accuracy control values is set to from 15 to 125 in the accuracy control graph divided into the plurality of plot areas, so that the user can set the number of plots of the accuracy control values based on characteristics of the accuracy control sample.

A specimen processing system according to a fifth aspect is the specimen processing system according to the second aspect, in which in a plurality of divided plot areas in the accuracy control graph, the display control unit assigns the plot area on a daily basis to accuracy control values obtained on a past analysis date and plots an average value of the accuracy control values obtained on the past analysis date at one point, and assigns the plot area on a time basis to the accuracy control value obtained today and plots the accuracy control value in the plot area.

In the specimen processing system according to the fifth aspect, the display control unit assigns a plot area on a daily basis to the accuracy control value obtained on the past analysis date in a plurality of divided plot areas in the accuracy control graph, and plots an average value of the accuracy control values obtained on the analysis date at one point. With respect to the accuracy control values obtained today, the display control unit assigns a plot area on a time basis and plots the accuracy control value in the plot area. Thus, in the present aspect, the user can easily visually recognize the transition of the accuracy control values in the past and the transition of the accuracy control values of today.

### Advantageous Effects of Invention

As described above, the specimen processing system according to the disclosure has an excellent effect that tendencies of accuracy control values of an accuracy control sample to be used for future accuracy control can be grasped in advance.

### BRIEF DESCRIPTION OF DRAWINGS

Fig. 1 is a schematic view illustrating a configuration of a specimen processing system according to the present embodiment.
Fig. 2 is a plan view schematically illustrating a configuration of a specimen processing apparatus according to the present embodiment.
Fig. 3 is a plan view schematically illustrating a configuration of a reagent chamber provided in the specimen processing apparatus according to the present embodiment.
Fig. 4 is a block diagram illustrating a hardware configuration of the specimen processing apparatus according to the present embodiment.
Fig. 5 is a block diagram illustrating a functional configuration of a control device mounted on the specimen processing apparatus according to the present embodiment.
Fig. 6 is a block diagram illustrating a hardware configuration of a terminal constituting a part of the specimen processing system according to the present embodiment.
Fig. 7 is a block diagram illustrating a functional configuration of the terminal constituting the part of the specimen processing system according to the present embodiment.
Fig. 8 is an image diagram illustrating a sample of a reagent information display screen displayed on the terminal constituting the part of the specimen processing system according to the present embodiment.
Fig. 9 is an image diagram illustrating a sample of an accuracy control setting screen displayed on the terminal constituting the part of the specimen processing system according to the present embodiment.
Fig. 10 is an image diagram illustrating a sample of an accuracy control data display screen displayed on the terminal constituting the part of the specimen processing system according to the present embodiment.
Fig. 11 is an image diagram illustrating a sample of the accuracy control data display screen displayed on the terminal constituting the part of the specimen processing system according to the present embodiment.

### DESCRIPTION OF EMBODIMENTS

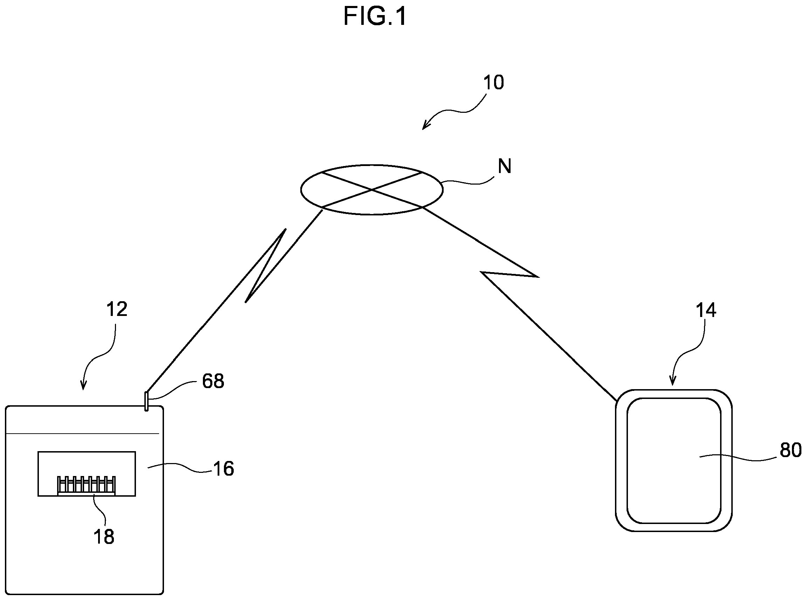

Hereinafter, an example of an embodiment of a specimen processing system according to the disclosure will be described with reference to Figs. 1 to 11. As illustrated in Fig. 1, a specimen processing system 10 according to the present embodiment includes a specimen processing apparatus 12, a terminal 14 used by a user, and a network N that communicably connects these components.

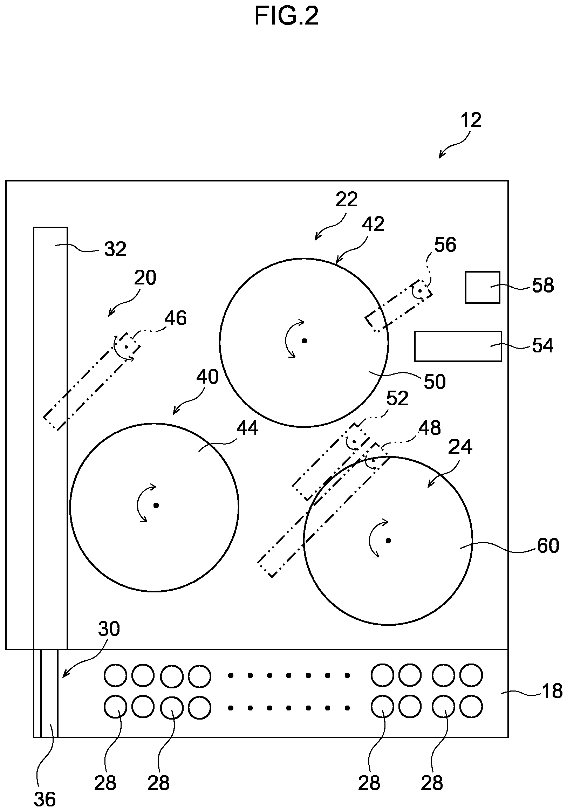

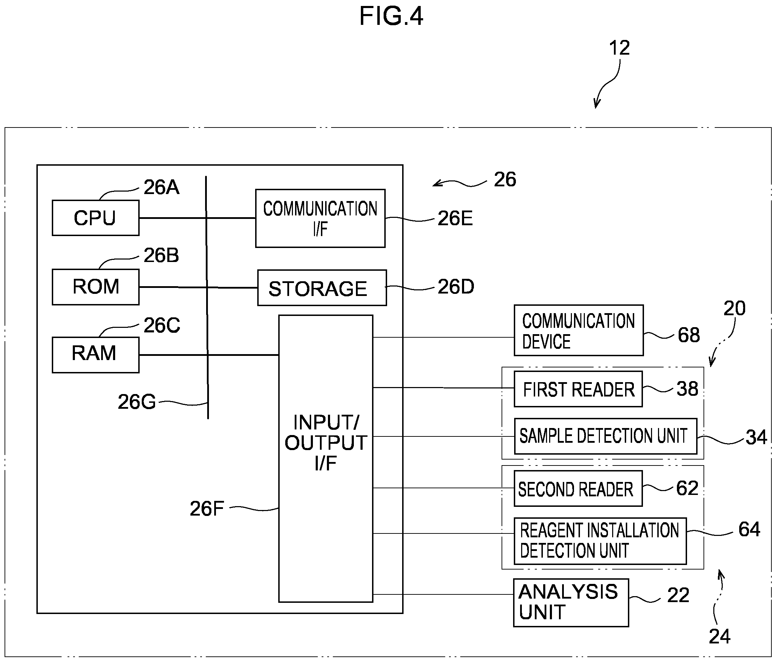

As also illustrated in Figs. 2 and 4, the specimen processing apparatus 12 includes a housing unit 16 constituting an outer shell thereof, a sample installation unit 18, a conveyance device 20, an analysis unit 22, a reagent management device 24 used to manage a reagent, and a control device 26 used to control various devices in the specimen processing apparatus 12.

The sample installation unit 18 is provided on the front side of the housing unit 16, and the sample installation unit 18 stores a plurality of specimen containers 28 in which various specimens such as blood and urine and a predetermined sample are put. A seal (not illustrated) on which an identifier such as a barcode or a QR code (registered trademark) indicating various types of information regarding the specimen injected into the specimen container 28 is printed is attached to the specimen container 28.

The conveyance device 20 includes a sampler unit 30, a conveyance rail 32, and a sample detection unit 34. The sampler unit 30 is movable along a moving rail (not illustrated) provided in the housing unit 16, and includes a gripping unit 36 capable of gripping the specimen container 28 and a first reader 38 capable of reading an identifier of the specimen container 28.

The sampler unit 30 can transport a predetermined specimen container 28 to the conveyance rail 32 in a state of being gripped by the gripping unit 36 based on an instruction signal transmitted from the control device 26. Upon receiving the instruction signal from the sample detection unit 34, the sampler unit 30 moves to a predetermined position of the specimen container 28, and transmits information regarding the sample in the specimen container 28 obtained from the identifier of the specimen container 28 to the control device 26 via the first reader 38.

The conveyance rail 32 transports the specimen container 28 to a predetermined position in the specimen processing apparatus 12 based on an instruction signal transmitted from the control device 26. When a specimen of an amount necessary for measurement is collected from the specimen container 28, the specimen container 28 is returned to a predetermined position of the sample installation unit 18 by the sampler unit 30 and the conveyance rail 32.

The sample detection unit 34 is provided in the sample installation unit 18, and includes a weight sensor, a contact sensor, and the like. When the specimen container 28 is newly stored in the sample installation unit 18, the sample detection unit 34 detects the specimen container 28 by the sensor, and transmits a command signal including position information of the specimen container 28 to the sampler unit 30.

The sampler unit 30 that has received the command signal from the sample detection unit 34 moves to the position of the newly stored specimen container 28, and transmits information regarding the specimen in the specimen container 28 and position information of the specimen container 28 to the control device 26.

The analysis unit 22 includes a colorimetric measurement unit 40 and a coagulation measurement unit 42. Specifically, the colorimetric measurement unit 40 includes a colorimetric reaction table 44 having a circular shape in plan view and provided with a plurality of colorimetric measurement ports (not illustrated), and the colorimetric reaction table 44 is rotatable clockwise and counterclockwise by an actuator (not illustrated) that operates based on an instruction signal transmitted from the control device 26.

Then, in each colorimetric measurement port of the colorimetric reaction table 44, a reaction container, for example, a cuvette is stored, and a specimen is injected from the specimen container 28 conveyed by the conveyance device 20 into the reaction container via the dispensing device 46, and a reagent stored in a reagent cooling storage 60 described later is injected into the reaction container via the dispensing device 48.

The colorimetric measurement unit 40 irradiates the reaction container into which the specimen and the reagent are injected with light of a specific wavelength to measure a color tone change of the mixed liquid of the specimen and the reagent, for example, absorbance, and calculates an activity value of a substance to be measured from the absorbance after a predetermined time or the amount of change in absorbance within a predetermined time.

The coagulation measurement unit 42 includes a coagulation reaction table 50 having a circular shape in plan view, and a plurality of coagulation measurement ports (not illustrated) is provided in the coagulation reaction table 50. The coagulation reaction table 50 is rotatable clockwise and counterclockwise by an actuator (not illustrated) that operates based on an instruction signal transmitted from the control device 26.

A reaction container is housed in each coagulation measurement port of the coagulation reaction table 50, and a specimen is injected into the reaction container from the specimen container 28 conveyed by the conveyance device 20 via the dispensing device 46, and a reagent stored in the reagent cooling storage 60 is injected into the reaction container via the dispensing device 52. The reaction container supplied from the reaction container supply unit 54 is supplied to each coagulation measurement port of the coagulation reaction table 50 via the reaction container transfer device 56. The used reaction container is recovered from the coagulation reaction table 50 by the reaction container transfer device 56 and discarded in the reaction container discard section 58.

The coagulation measurement unit 42 irradiates the reaction container into which the specimen and the reagent are injected with light, and detects a coagulation reaction or the like of a substance to be measured from a process of intensity change of scattered light.

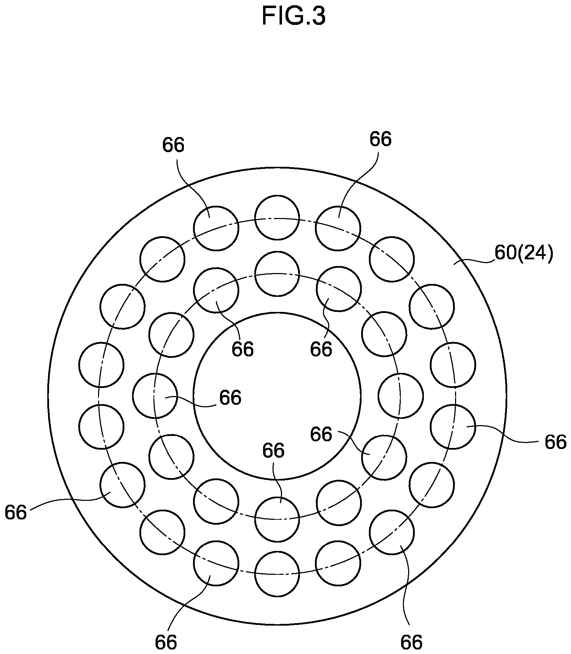

The reagent management device 24 includes the reagent cooling storage 60, a second reader 62, and a reagent installation detection unit 64. As also illustrated in Fig. 3, the reagent cooling storage 60 has a circular shape in plan view, and can cool a plurality of reagent containers 66 into which reagents used for various analyses of a specimen are injected. The reagent cooling storage 60 is rotatable clockwise and counterclockwise by an actuator (not illustrated) that operates based on an instruction signal transmitted from the control device 26.

A seal (not illustrated) on which an identifier such as a barcode or a QR code indicating various types of information regarding the reagent injected into the reagent container 66 is printed is attached to the reagent container 66.

The information of the reagent indicated by the identifier includes, for example, the lot number of the reagent, the serial number of the reagent, the type of the reagent, a measurement item in which the reagent is used, the type of the reagent container, the expiration date of the reagent, the capacity of the reagent container, the number of analyzable times, and an ISI value of the reagent.

The second reader 62 can acquire information regarding the reagent in the reagent container 66 by reading the identifier of the seal attached to the reagent container 66. The second reader 62 can transmit and receive various types of information to and from the control device 26, and can transmit information regarding the reagent in the reagent container 66 to the control device 26.

The reagent installation detection unit 64 is provided in the reagent cooling storage 60, and includes a weight sensor, a contact sensor, and the like. When the reagent container 66 is newly stored in the reagent cooling storage 60, the reagent installation detection unit 64 detects the reagent container 66 by the sensor, and transmits the position information of the reagent container 66 to the control device 26.

The control device 26 stores the information regarding the reagent in the reagent container 66 transmitted from the second reader 62 and the position information of the reagent container 66 transmitted from the reagent installation detection unit 64 in association with each other.

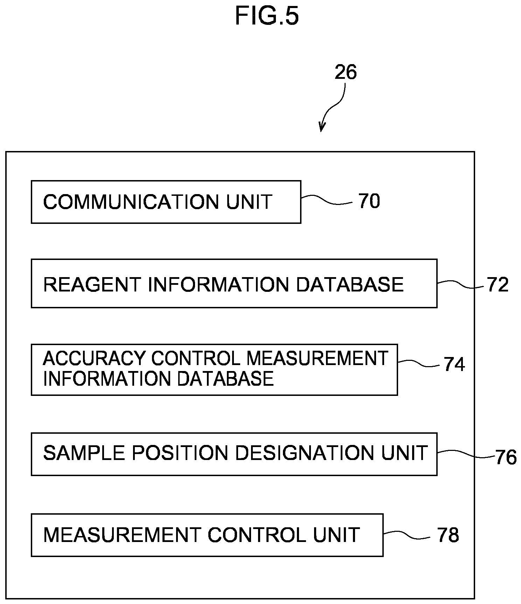

Next, a configuration of the control device 26 will be described with reference to Figs. 4 and 5. The control device 26 includes a CPU 26A which is an example of a processor, that is, a Central Processing Unit, a ROM 26B, that is, a Read Only Memory, a RAM 26C, that is, a Random Access Memory, a storage 26D, and a communication I/F 26E, that is, an Inter Face and an input/output I/F 26F. The CPU 26A, the ROM 26B, the RAM 26C, the storage 26D, the communication I/F 26E, and the input/output I/F 26F are communicably connected to each other via a bus 26G.

The CPU 26A is a central processing unit, and can execute various programs related to various controls of the specimen processing apparatus 12. Specifically, the CPU 26A can read a program from the ROM 26B and execute the program using the RAM 26C as a work area. Then, the execution program stored in the ROM 26B is read and executed by the CPU 26A, whereby the control device 26 can exhibit various functions as described later.

The storage 26D includes an HDD, that is, a hard disk drive or an SSD, that is, a solid state drive, and stores various programs including an operating system and various data. The storage 26D can store various types of information and the like regarding the specimen and the reagent.

The communication I/F 26E is an interface used for connection between the control device 26 and the network N, and the communication I/F 26E can communicate with the terminal 14 and the like via the communication device 68 including various antennas and the like connected to the input/output I/F 26F and the network N. For this interface, for example, a communication standard such as Wi-Fi (registered trademark) is used.

The input/output I/F 26F is an interface for the control device 26 to communicate with each device mounted on the specimen processing apparatus 12. The control device 26 is communicably connected to the conveyance device 20, the analysis unit 22, and the reagent management device 24 via the input/output I/F 26F. These devices may be directly connected to the bus 26G.

Next, a functional configuration of the control device 26 will be described. The control device 26 functions as an assembly of a communication unit 70, a reagent information database 72, an accuracy control measurement information database 74 as an accuracy control information storage unit, a sample position designation unit 76, and a measurement control unit 78 by the CPU 26A reading the execution program stored in the ROM 26B and executing the read execution program.

As will be described later, the communication unit 70 can transmit and receive various types of information to and from the terminal 14 via the network N.

The reagent information database 72 is a database that stores reagent information regarding each reagent stored in the reagent cooling storage 60. As an example, the reagent information database 72 stores information regarding the reagent in the reagent container 66 read from the identifier of the reagent container by the second reader 62, the storage 26D, that is, detailed information of the reagent regarding each reagent used in the specimen processing system 10 stored in advance in the specimen processing system 10, and the position information of the reagent container 66 transmitted from the reagent installation detection unit 64 in association with each other.

The accuracy control measurement information database 74 is a database that stores information regarding accuracy control measurement corresponding to each reagent used in the specimen processing system 10. The accuracy control measurement means measurement for evaluating reliability of the specimen processing apparatus 12 in measurement of a specimen. In the accuracy control measurement, if the accuracy control measurement result of an accuracy control sample that can be regarded as having the same concentration of a measurement target substance in the same lot, that is, the so-called control sample falls within an allowable range before and after the measurement of the specimen, it is regarded that the reliability of the specimen processing apparatus 12 is ensured.

Specifically, the accuracy control measurement information database 74 stores, for each reagent used in the specimen processing system 10, a measurement item in which the reagent is used, information regarding a control sample used for accuracy control measurement of the reagent, and a measurement result of the accuracy control measurement described later. Examples of the information regarding the control sample include a sample name, position information in the sample installation unit 18, a remaining amount, and a production lot number.

It is also possible to set, as a function of the control device 26, an integrated database in which a part or all of the information stored in the reagent information database 72 and a part or all of the information stored in the accuracy control measurement information database 74 are integrated and operated.

As described later, the sample position designation unit 76 selects a control sample corresponding to the reagent designated by the terminal 14 based on the data of the accuracy control measurement information database 74. Then, based on the state of the sample installation unit 18 obtained from the sample detection unit 34, the specified position information for storing the control sample in the sample installation unit 18 is transmitted to the terminal 14.

The measurement control unit 78 performs arithmetic processing on detection results of the colorimetric measurement unit 40 and the coagulation measurement unit 42 based on the calibration curve or the like stored in the storage 26D, calculates the concentration or reaction time of the substance to be analyzed in the specimen, and stores the results.

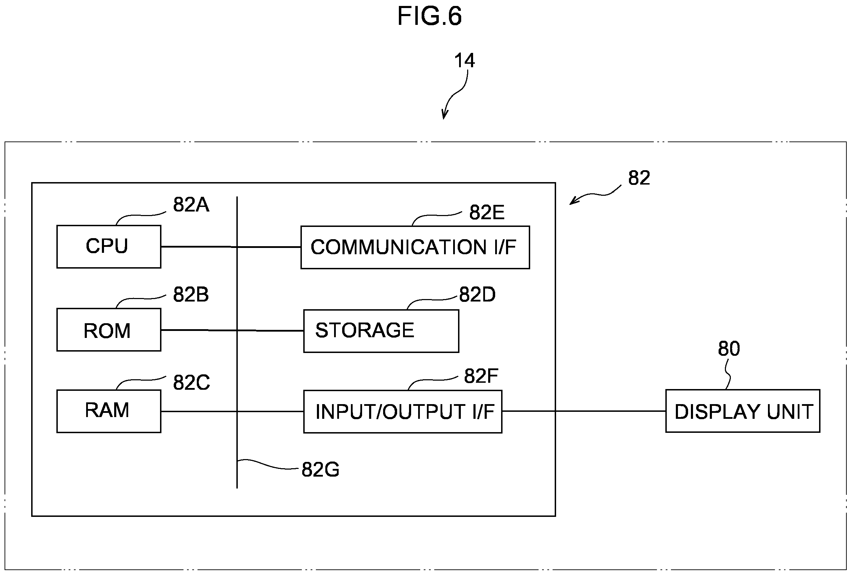

As illustrated in Fig. 6, the terminal 14 includes, as an example, a touch display 80 as a display unit capable of displaying various information to the user and detecting an operation input by the user, and a control device 82. The terminal 14 can communicate with the specimen processing apparatus 12 and the like via the network N. Various devices such as a tablet can be employed as the terminal 14.

The control device 82 includes a CPU 82A, a ROM 82B, a RAM 82C, a storage 82D, a communication I/F 82E, and an input/output I/F 82F. The CPU 82A, the ROM 82B, the RAM 82C, the storage 82D, the communication I/F 82E, and the input/output I/F 82F are communicably connected to each other via a bus 82G. The CPU 82A, the ROM 82B, the RAM 82C, the storage 82D, the communication I/F 82E, and the input/output I/F 82F basically have functions similar to those of the control device 26 described above.

In the present embodiment, various execution programs stored in the ROM 82B are read and executed by the CPU 82A, whereby various information can be displayed on the touch display 80 based on various reagents, data related to various specimens, and the like acquired from the specimen processing apparatus 12 and stored in the storage 82D. The touch display 80 is connected to the control device 82 via the input/output I/F 82F so as to be able to communicate with each other.

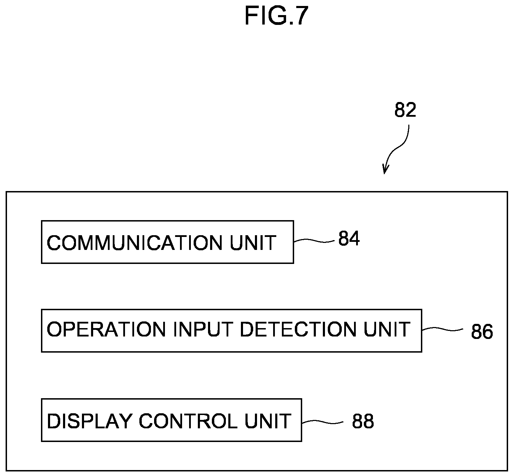

Specifically, as illustrated in Fig. 7, a control device 82 functions as an assembly of a communication unit 84, an operation input detection unit 86, and a display control unit 88 by the CPU 82A reading the execution program stored in the ROM 82B and executing the read execution program.

The communication unit 84 can transmit and receive various types of information to and from the control device 26 via the network N. Specifically, the communication unit 84 can acquire various data from the reagent information database 72 and the accuracy control measurement information database 74.

The operation input detection unit 86 detects an input made by the user to the touch display 80 and transmits the input to the display control unit 88.

The display control unit 88 displays various screens and buttons related to the operation of the specimen processing apparatus 12 on the touch display 80, and reflects the user's input to the touch display 80 on the screens and buttons. The display control unit 88 displays Information obtained from the reagent information database 72 and the accuracy control measurement information database 74 via the communication unit 84 is displayed on the touch display 80.

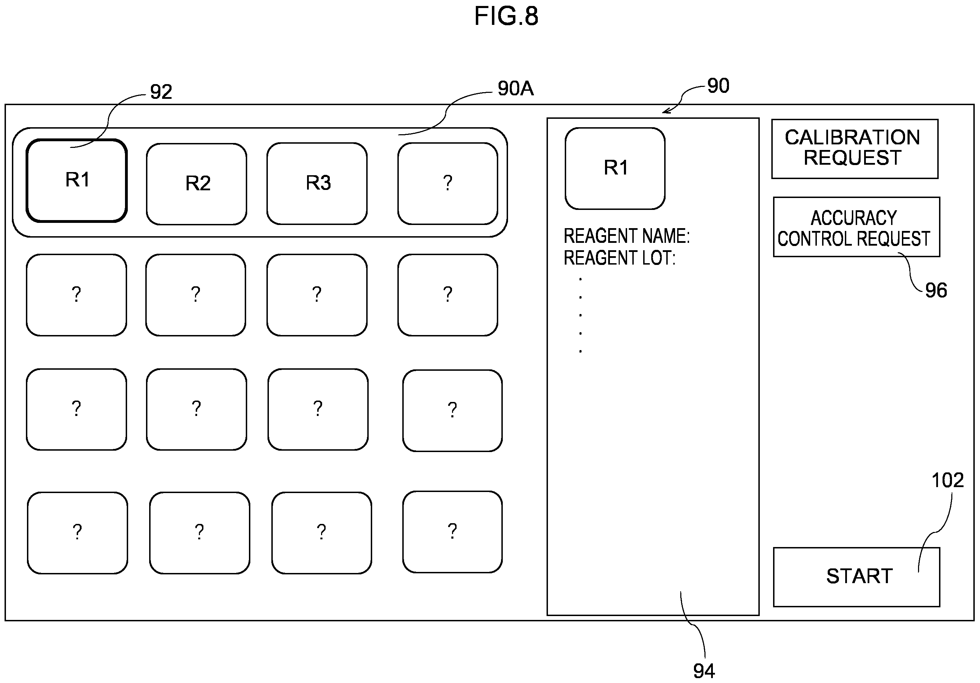

Specifically, when the user makes an input to a reagent storage tab (not illustrated) displayed on the touch display 80, a reagent information display screen 90 is displayed on the touch display 80 as illustrated in Fig. 8.

The reagent information display screen 90 includes a reagent recognition mark display region 90A, and in the reagent recognition mark display region 90A, a reagent recognition mark 92 corresponding to a reagent is displayed so as to correspond to an arrangement position of the reagent in the reagent cooling storage 60 on each reagent stored in the reagent cooling storage 60. In the reagent recognition mark display region 90A, as an example, at a portion where the reagent is not disposed in the reagent cooling storage 60 or a position of the reagent where the second reader 62 has not been able to acquire the information regarding the reagent, a ? mark is displayed.

When the user makes an input to the reagent recognition mark 92 on the reagent information display screen 90, a reagent information display screen 94 for displaying reagent information of the reagent corresponding to the reagent recognition mark 92 is displayed on the reagent information display screen 90.

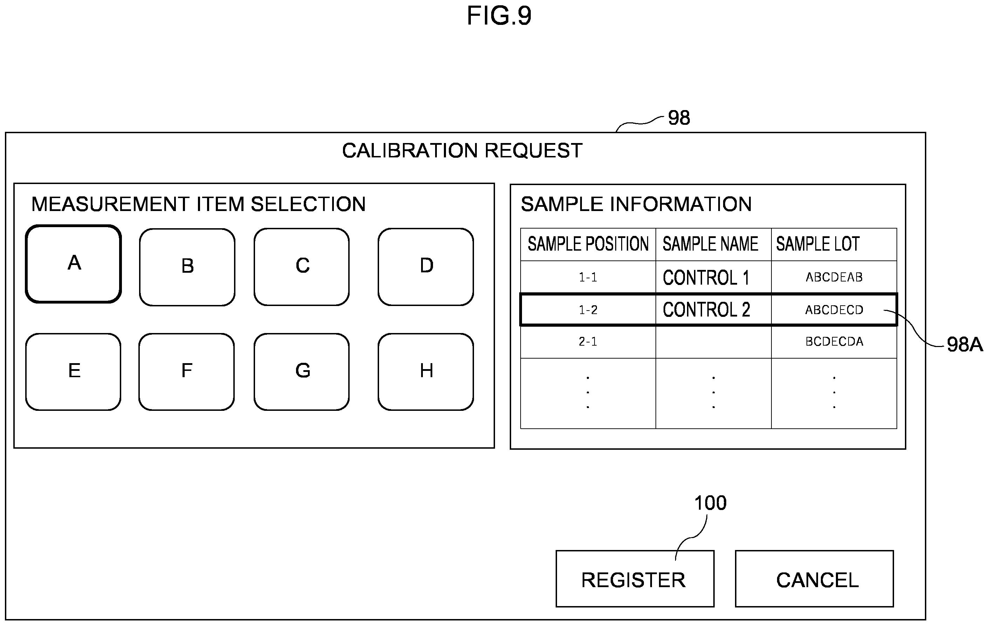

An accuracy control request button 96 is displayed on the reagent information display screen 90. Then, when the user makes an input to the accuracy control request button 96 in a state where the reagent recognition mark 92 is selected, that is, in a state where the reagent recognition mark 92 is highlighted, as illustrated in Fig. 9, an accuracy control setting screen 98 related to the accuracy control measurement of the reagent corresponding to the reagent recognition mark 92 is displayed on the reagent information display screen 90.

On the accuracy control setting screen 98, the measurement item of the specimen in which the reagent corresponding to the reagent recognition mark 92 is used, and an information display field 98A regarding a control sample necessary for accuracy control measurement regarding the measurement item are highlighted.

Specifically, the accuracy control setting screen 98 includes, as the information regarding the control sample, the position where the control sample is disposed in the sample installation unit 18, the sample name of the control sample, the production lot number of the control sample, and the type of container of the control sample.

Then, the user disposes the control sample at a predetermined position designated on the accuracy control setting screen 98, and when the user makes an input to a registration button 100, the accuracy control setting screen 98 is closed. In this state, when the user makes an input to a start button 102, the accuracy control measurement is started.

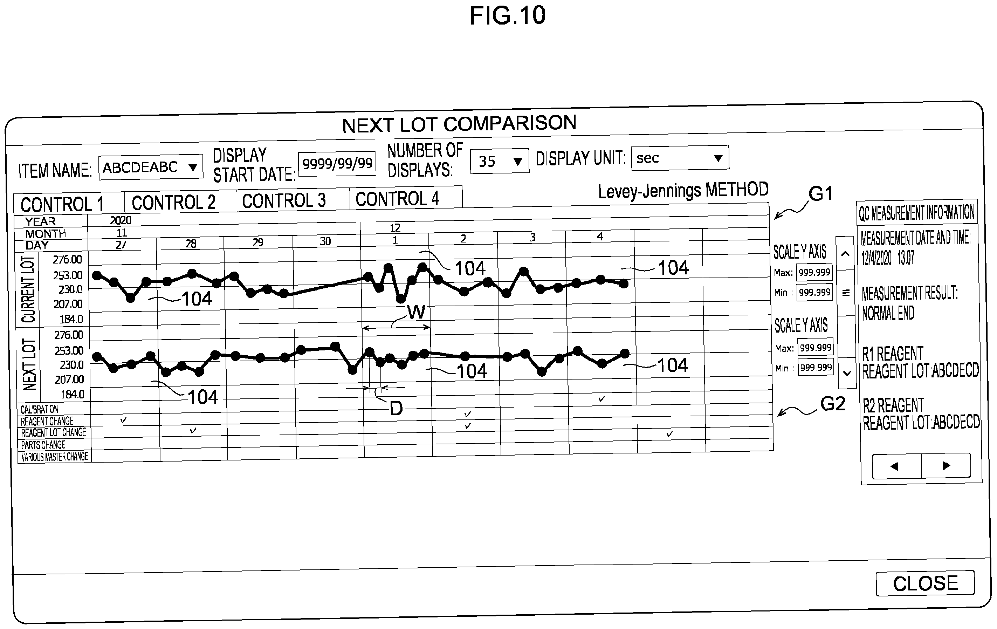

Then, the measurement result of the accuracy control measurement performed as described above, that is, the accuracy control value is stored in the accuracy control measurement information database 74 in association with the time series order, more specifically, the date and time when the accuracy control measurement was performed, for each type of control sample and each production lot number. As illustrated in Fig. 10, in the accuracy control measurement information database 74, these accuracy control values are stored as an accuracy control graph by the Levey-Jennings method.

Specifically, in the accuracy control measurement information database 74, as an example, an accuracy control graph G1 obtained by plotting accuracy control values of a currently used control sample in time series order and an accuracy control graph G2 obtained by plotting accuracy control values of a control sample of the same type as the currently used control sample and of a different production lot number therefrom in time series order are generated.

In the present embodiment, when the user makes an input to an accuracy control tab (not illustrated) displayed on the touch display 80 of the terminal 14, the accuracy control graph G1 and the accuracy control graph G2 are displayed.

The accuracy control graph G1 and the accuracy control graph G2 are divided into a plurality of plot areas 104 in a time series direction, and one or a plurality of accuracy control values are plotted in these plot areas 104.

In the accuracy control graph G1 and the accuracy control graph G2, the plot area 104 is divided in units of one day, and the accuracy control value obtained on each analysis day is plotted in the plot area 104. Nothing is plotted in the plot area 104 on a day on which the accuracy control measurement has not been performed.

In the present embodiment, the size of the plot area 104 in the time series direction, that is, the width W is set to a size in which it is possible to ensure a predetermined interval D between plots in the plot area 104 having the largest number of accuracy control values.

Furthermore, in the present embodiment, the number of plots of the accuracy control value displayed in the plot area 104, that is, the maximum number of plots is set to from 15 to 125.

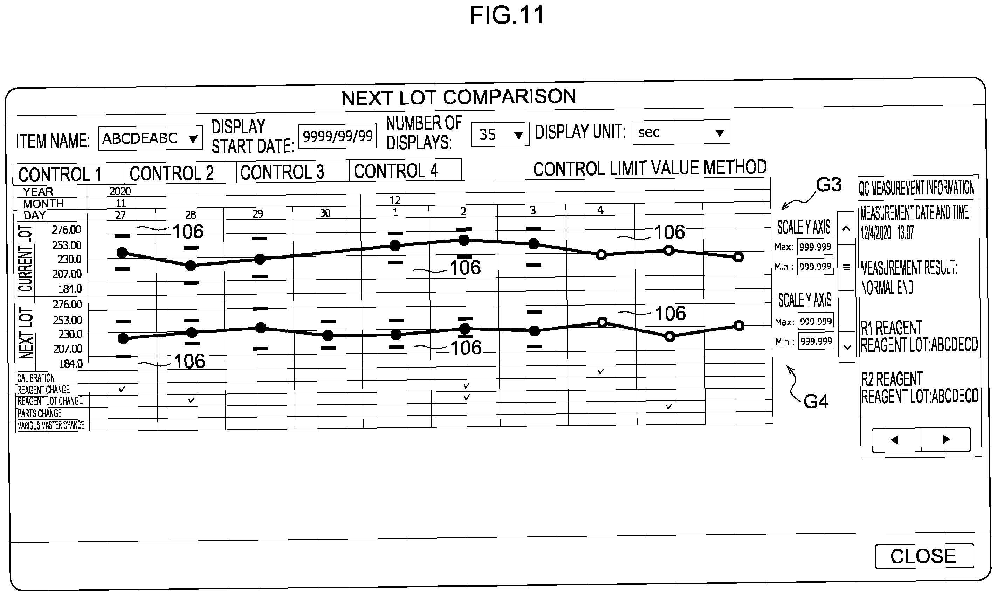

As illustrated in Fig. 11, in the accuracy control measurement information database 74, the accuracy control value obtained by the accuracy control measurement is also stored as an accuracy control graph by a control limit value method.

Specifically, in the accuracy control measurement information database 74, as an example, an accuracy control graph G3 in which the maximum value, the minimum value, and the average value of the accuracy control values obtained on the past analysis date in the currently used control sample are plotted for each analysis day, and the accuracy control value obtained on the present day, that is, the display date is plotted in time series order, and an accuracy control graph G4 in which the maximum value, the minimum value, and the average value of the accuracy control values obtained on the past analysis date in the control sample of the same type as the currently used control sample and a different production lot number are plotted for each analysis day, and the accuracy control value obtained today is plotted in time series order are generated.

**In** the present embodiment, in a state where the accuracy control graph G1 and the accuracy control graph G2 are displayed on the touch display 80, when the user makes an input to a display switching button (not illustrated), the accuracy control graph G3 and the accuracy control graph G4 are displayed. **In** a state where the accuracy control graph G3 and the accuracy control graph G4 are displayed on the touch display 80, when the user makes an input to the display switching button, the display screen is switched to the display screen of the accuracy control graph G1 and the accuracy control graph G2.

Similarly to the accuracy control graph G1 and the accuracy control graph G2, the accuracy control graph G3 and the accuracy control graph G4 are divided into a plurality of plot areas 106 in the time system column direction. Then, in the portion displaying the accuracy control value obtained in the past in the accuracy control graph G3 and the accuracy control graph G4, the plot area 106 is divided in units of one day, and in the portion displaying the accuracy control value obtained today in the accuracy control graph G3 and the accuracy control graph G4, the plot area 106 is divided in units of one hour, for example.

In the accuracy control graph G3 and the accuracy control graph G4, the maximum value and the average value of the accuracy control values obtained on each analysis day are displayed as bars and the average value is plotted with black circles in the plot area 106 of the past one day. In the plot area 106 of the accuracy control graph G3 and the accuracy control graph G4 on the display date, the accuracy control value obtained on the display date is plotted with a white circle. At this time, it is preferable that all accuracy control values obtained on the display date are plotted. Nothing is plotted in the plot area 106 in a period in which the accuracy control measurement has not been performed.

### Operations and effects of present embodiment

Next, functions and effects of the present embodiment will be described.

In the present embodiment, as illustrated in Fig. 2, the analysis unit 22 is provided, and components in a specimen are analyzed by the analysis unit 22.

Incidentally, in order to ensure the reliability of the specimen processing apparatus 12 and hence the specimen processing system 10, it is necessary to measure the accuracy control sample containing the measurement target substance in the specimen, that is, perform the accuracy control by the analysis unit 22 before and after the analysis of the specimen.

Then, the concentration of the predetermined measurement target substance in the accuracy control sample differs for each production lot, and thus, when the production lot number of the accuracy control sample currently used for the accuracy control is different from the production lot number of the accuracy control sample scheduled to be used for the accuracy control, it is preferable that tendencies of the accuracy control values of the accuracy control sample to be used for the future accuracy control can be grasped in advance.

Here, in the present embodiment, the accuracy control measurement information database 74 stores a plurality of pieces of accuracy control data that is time series data of the accuracy control value obtained by analyzing the accuracy control sample by the analysis unit 22, and the accuracy control data is displayed on the touch display 80 by the display control unit 88 as illustrated in Figs. 10 and 11.

Specifically, the display control unit 88 simultaneously displays the accuracy control data of a plurality of accuracy control samples of the same type and different production lot numbers on the touch display 80 based on the accuracy control data stored in the accuracy control measurement information database 74. Thus, in the present embodiment, the user can compare the accuracy control data of a plurality of accuracy control samples of the same type and different production lot numbers.

For example, the user can compare the accuracy control sample currently used for accuracy control with the accuracy control sample scheduled to be used for accuracy control and currently used and an accuracy control sample of a different production lot number. As a result, in a case in which the accuracy control is performed using the accuracy control sample that is scheduled to be used for the accuracy control and has a production lot number different from that of the currently used accuracy control sample, the user can accurately grasp the normal range of the accuracy control value, that is, the upper limit value and the lower limit value, and thus the accuracy of the accuracy control can be improved.

In the present embodiment, the accuracy control data includes a plurality of accuracy control values obtained on different analysis dates, and the display control unit 88 displays the accuracy control graph G1 and the accuracy control graph G2 obtained by plotting the accuracy control values on the touch display 80 as the accuracy control data. Thus, in the present embodiment, the user can observe changes in the accuracy control values on a plurality of analysis days by viewing the accuracy control data.

In the present embodiment, by plotting the accuracy control values obtained in the analysis date, that is, one day in time series order for each of the plurality of plot areas 104, it is possible to observe changes in the accuracy control values in more detail.

By setting the size W of the plot area 104 in the time series direction to a size in which the interval between the plots in the plot area 104 having the largest number of plots of the accuracy control values can ensure the predetermined interval D, it is possible to ensure the visibility of the accuracy control graph G1 and the accuracy control graph G2.

In the present embodiment, the number of plots of the accuracy control values is set to from 15 to 125 in the accuracy control graph G1 and the accuracy control graph G2 divided into the plurality of plot areas 104, so that the user can set the number of plots of the accuracy control values based on characteristics of the accuracy control sample.

In addition, in the present embodiment, the display control unit 88 assigns the plot area 106 on a daily basis to the accuracy control value obtained on the past analysis date in the plurality of divided plot areas 106 in the accuracy control graph G3 and the accuracy control graph G4, and plots the average value of the accuracy control values obtained on this analysis date at one point. With respect to the accuracy control values obtained today, the display control unit 88 assigns the plot area 106 on a time basis, and plots all the accuracy control values one by one in the plot area 106. Thus, in the present embodiment, the user can easily visually recognize the transition of the accuracy control values in the past and the transition of the accuracy control values of today.

As described above, the specimen processing system 10 according to the present embodiment can grasp in advance tendencies of accuracy control values of an accuracy control sample to be used for future accuracy control.

### <Supplementary description of above embodiment>

(1) In the embodiment described above, the reagent information display screen 90 and the like are displayed on the touch display 80 of the terminal 14, but a configuration may be employed in which a touch display similar to the touch display 80 is provided in the specimen processing apparatus 12. The reagent information display screen 90 or the like may be displayed on a monitor of a computer communicable with the specimen processing apparatus 12.
(2) In the embodiment described above, a seal (not illustrated) on which an identifier indicating various types of information is printed is attached to the specimen container 28 or the like, but the present invention is not limited thereto. For example, a configuration may be employed in which an RF tag in which various types of information are stored, that is, a radio frequency tag is provided in the specimen container 28 or the like, and electronic information of the RF tag is read and electronic information is written in the RF tag by a reader/writer provided in the specimen processing apparatus 12.

## Claims

1. A specimen processing system comprising:
an analysis unit that analyzes a component in a specimen;
an accuracy control information storage unit that stores a plurality of pieces of accuracy control data that is time series data of an accuracy control value obtained by analyzing an accuracy control sample by the analysis unit; and
a display control unit that causes a display unit to display the accuracy control data,
wherein the display control unit simultaneously displays the accuracy control data of a plurality of accuracy control samples of a same type and different production lot numbers on the display unit based on the accuracy control data stored in the accuracy control information storage unit.

2. The specimen processing system according to claim 1, wherein:
the accuracy control data includes a single or a plurality of the accuracy control values obtained on different analysis dates, and
the display control unit displays an accuracy control graph obtained by plotting the accuracy control values as the accuracy control data.

3. The specimen processing system according to claim 2, wherein:
the display control unit plots a single or a plurality of the accuracy control values obtained on the analysis dates for each of a plurality of plot areas divided in the accuracy control graph in time series order, and
a size in a time series direction of the plurality of plot areas is set to a size at which it is possible to ensure a predetermined interval for an interval for each of plots in a plot area having a largest number of the plots of the accuracy control values.

4. The specimen processing system according to claim 3, wherein
a number of the plots of the accuracy control values displayed in the plot area is set to from 15 to 125.

5. The specimen processing system according to claim 2, wherein
in a plurality of divided plot areas in the accuracy control graph, the display control unit assigns the plot area on a daily basis to accuracy control values obtained on a past analysis date and plots an average value of the accuracy control values obtained on the past analysis date at one point, and assigns the plot area on a time basis to an accuracy control value obtained today and plots the accuracy control value in the plot area.
